# EUROPEAN PATENT SPECIFICATION

(11) **EP 4 009 120 B1**
(45) Date of publication and mention of the grant of the patent: **25.02.2026**
(21) Application number: 21211012.6
(22) Date of filing: 29.11.2021
(51) Int. Cl.: G04G 17/04, G04G 21/02

(54) **DETECTION DEVICE AND BODY-WORN DEVICE**
DETEKTIONSVORRICHTUNG UND KÖRPERGETRAGENE VORRICHTUNG
DISPOSITIF DE DÉTECTION ET DISPOSITIF PORTÉ SUR LE CORPS

(30) Priority: 03.12.2020 JP 2020200944; 25.03.2021 JP 2021051244
(43) Date of publication of application: 08.06.2022
(73) Proprietor: Casio Computer Co., Ltd., Tokyo 151-8543 (JP)
(72) Inventor: Ohmura, Akihisa, Hamura-shi, 205-8555 (JP); Otsubo, Hiroaki, Hamura-shi, 205-8555 (JP)
(74) Representative: Grünecker Patent- und Rechtsanwälte PartG mbB

(56) References cited:
- US-A1- 2007 191 718
- US-A1- 2018 348 048
- US-A1- 2019 082 985

## Description

### BACKGROUND

### 1. Technical Field

The present disclosure relates to a detection device that detects biological information such as a pulse, and a body-worn device equipped with the detection device.

### 2. Description of the Related Art

A detection device as described in the preamble of claim 1 is already known from US 2018/348 048 A1. Further detection devices are known from US 2007/191 718 A1 and US 2019/082 985 A1. For example, a detection device for detecting biological information such as a pulse is known which is provided on a back cover attached to the lower part of a case that is called a housing and worn on a part of a body such as an arm, as described in Japanese Patent Application Laid-Open (Kokai) Publication No. JP 2005-270543 A.

It is an object of the present invention to provide a detection device which has an improved sensor protection in case of shocks or impacts and a body-worn device comprising such a detection device.

### SUMMARY

The above and other objects of the invention are achieved by the detection device according to claim 1 and the body-worn device according to claim 8. Preferred embodiments are claimed in the dependent claims. One embodiment is a detection device comprising: a back cover which is provided with a light transmissive transparent portion; a holder which is attached to an arrangement recess section of the back cover and positioned opposing the transparent portion; a circuit board which is arranged between the back cover and the holder; an optical sensor which is provided on one surface of the circuit board opposing the transparent portion to detect light; and resilient energizing members which are arranged in compressed states in areas between the undersurface of the holder and the upper surface of the holder circuit board, thereby forcing the circuit board and the optical
sensor toward the transparent portion. The energizing member are made of cushioning material or are spring members such as coil springs or plate springs, and arranged on an outer circumferential area which is on another surface opposite to the one surface of the circuit board and does not include an area corresponding to the optical sensor.

The above and further objects and novel features of the present invention will more fully appear from the following detailed description when the same is read in conjunction with the accompanying drawings. It is to be expressly understood, however, that the drawings are for the purpose of illustration only and are not intended as a definition of the limits of the invention.

### BRIEF DESCRIPTION OF THE DRAWINGS

FIG. 1 is an enlarged front view showing an embodiment of a body-worn device;
FIG. 2 is an enlarged perspective view showing the back surface side of the body-worn device shown in FIG. 1;
FIG. 3 is an enlarged planar view showing the inner surface side of the back cover of the body-worn device shown in FIG. 2;
FIG. 4 is an enlarged cross-sectional view showing a main portion of the back cover taken along the A-A arrow view in FIG. 3;
FIG. 5 is an enlarged cross-sectional view showing the main portion of the back cover taken along the B-B arrow view in FIG. 3;
FIG. 6 is an enlarged perspective view showing a state where a holder for a detection device shown in FIG. 4 and FIG. 5 is
   attached to the back cover;
FIG. 7 is an enlarged rear view showing the inner surface side of the holder of FIG. 6;
FIG. 8 is an enlarged perspective view showing the undersurface side of a circuit board shown in FIG. 6; and
FIG. 9 is an enlarged perspective view showing the upper surface side of the circuit board shown in FIG. 6.

### DETAILED DESCRIPTION OF THE PREFERRED EMBODIMENTS

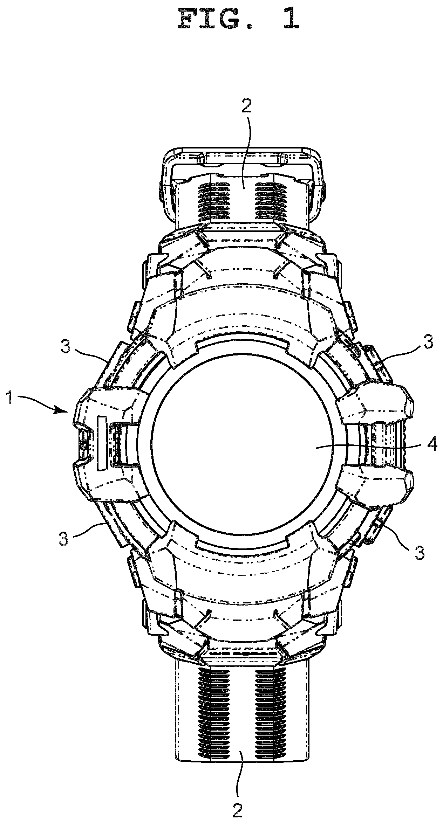

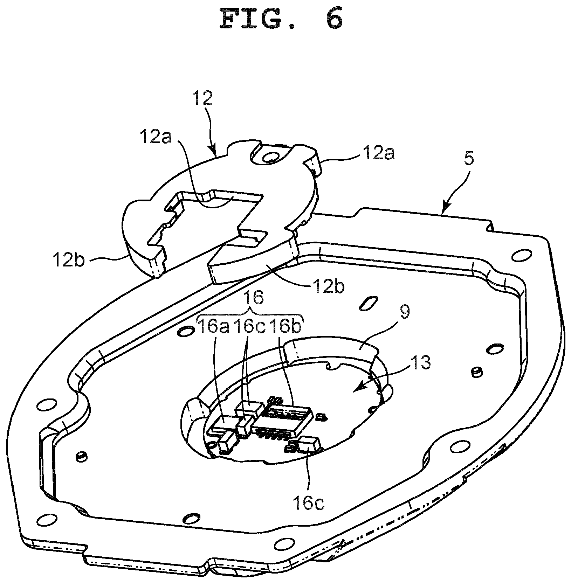

An embodiment where the present invention has been applied in a body-worn device will hereinafter be described with reference to FIG. 1 to FIG. 9.

This body-worn device is a wristwatch type device that is used with it being worn on an arm, and includes a case 1, as shown in FIG. 1. To outer circumferential portions of the case 1 on the twelve o'clock side and the six o'clock side, bands 2 are attached so that the case 1 can be worn on a part of a body such as an arm.

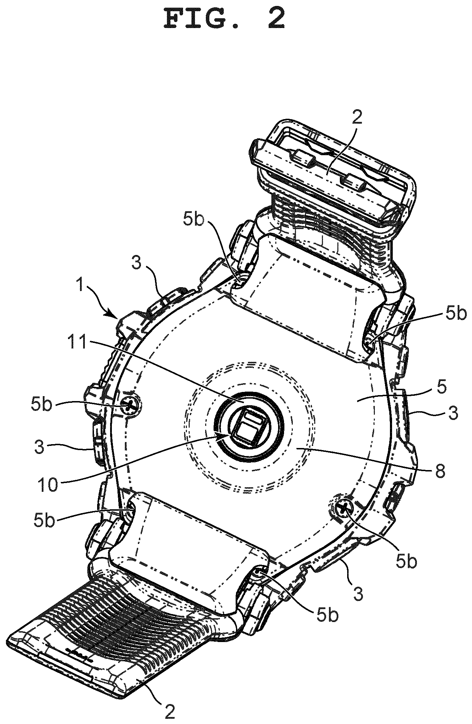

Also, on outer circumferential portions of the case 1 which do not correspond to the bands 2, push button switches 3 are provided, as shown in FIG. 1. In addition, to the upper opening of the case 1, a glass 4 is attached. Moreover, to the lower part of the case 1, a back cover 5 that is an exterior member is attached by a plurality of screws 5b, as shown in FIG. 2.

Also, inside the case 1, a module (not shown) is provided. This module includes various types of components, such as a display section for displaying biological information such as a pulse and various types of information regarding a time of day, a date, a day of the week, and the like, and a circuit section for electrically controlling the display section.

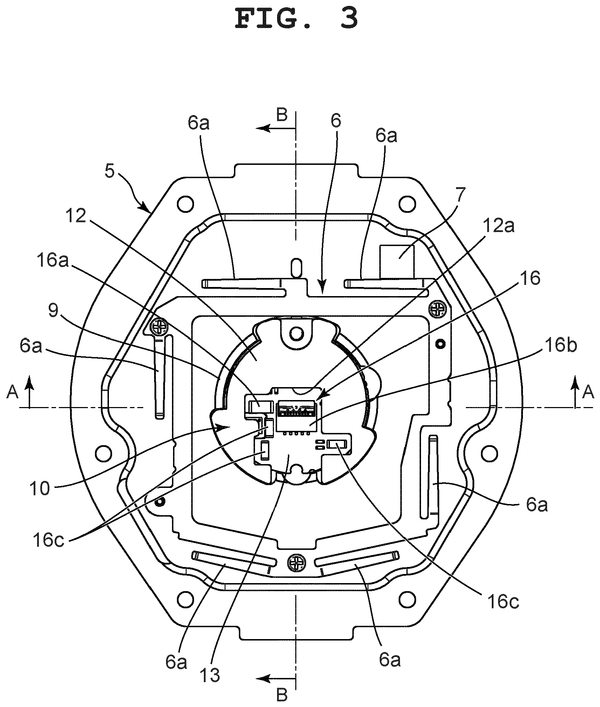

On the inner surface of the back cover 5, an electrode plate 6, a cushion member 7, and an insulation sheet (not shown) are provided, as shown in FIG. 3. The electrode plate 6 has a substantially quadrilateral frame shape, and is attached to the inner surface of the back cover 5 by screws. On the electrode plate 6, a plurality of contact pieces 6a is provided. As a result, the electrode plate 6 is structured such that, when the back cover 5 is attached to the lower part of the case 1, the plurality of contact pieces 6a comes in contact with the circuit section of the module in the case 1.

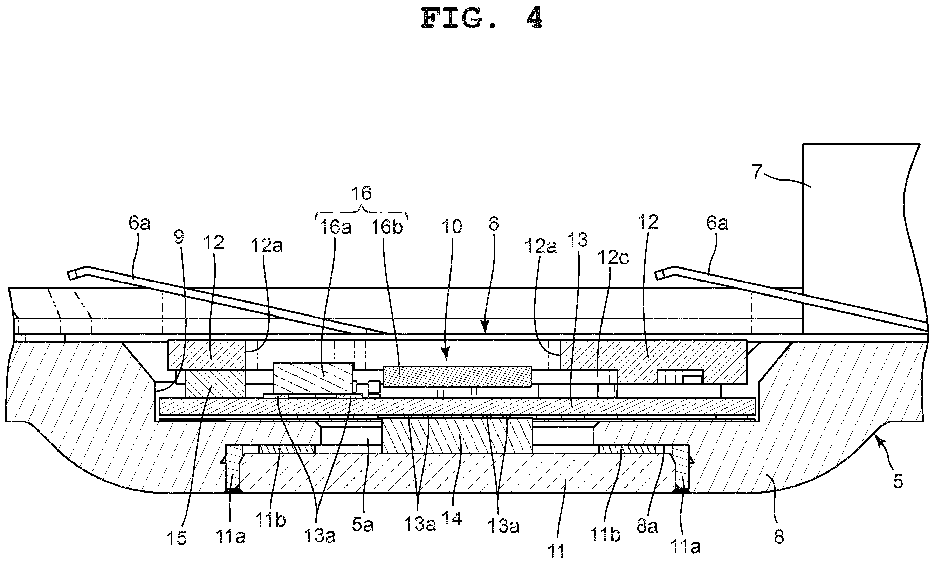

The cushion member 7 is structured to be provided on a portion of the inner surface of the back cover 5 and positioned outside the frame part of the electrode plate 6, and to elastically hold down the module in the case 1 when the back cover 5 is attached to the lower part of the case 1, as shown in FIG. 3 and FIG. 4. Although not shown in the drawings, the insulation sheet, which insulates a detection device 10 described later, is provided on a portion of the inner surface of the back cover 5 in a manner to be positioned in an area inside the frame part of the electrode plate 6 excluding areas corresponding to electronic components 16 of a later-described circuit board 13 of the detection device 10, and covers the detection device 10.

In the center area of the undersurface of the back cover 5, a projection section 8 having a substantially circular disk shape is provided, as shown in FIG. 2. This projection section 8 is structured to be pressed onto the skin of an arm when the case 1 is worn on the arm with the bands 2. In the inner surface of the projection section 8, an arrangement recess section 9 is provided in which the detection device 10 is arranged, as shown in FIG. 3 to FIG. 6.

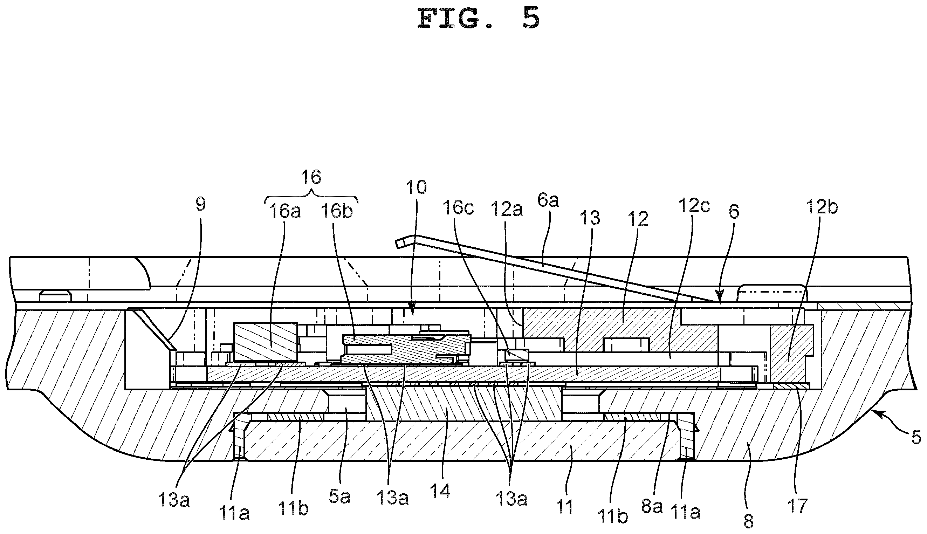

The detection device 10 is configured to detect biological information such as a pulse with the case 1 being worn on an arm by the bands 2, as shown in FIG. 1 and FIG. 2. More specifically, the detection device 10 includes a protective glass 11 which is a light transmissive transparent section and provided on the projection section 8 of the back cover 5, and a holder 12 which is attached to the arrangement recess section 9 of the back cover 5 in a manner to be positioned opposing the protective glass 11, as shown in FIG. 4 and FIG. 5.

This detection device 10 also includes the circuit board 13 which is arranged between the holder 12 and the bottom of the arrangement recess section 9 of the back cover 5, an optical sensor 14 which is provided on one surface of the circuit board 13 opposing the protective glass 11, that is, the undersurface of the circuit board 13 and detects light of a specific wavelength, and energizing members 15 which are arranged between the holder 12 and the circuit board 13 and force the circuit board 13 and the optical sensor 14 toward the protective glass 11, as shown in FIG. 4 and FIG. 5.

The protective glass 11 is fitted from below into an attachment recess section 8a in the projection section 8 of the back cover 5 via a waterproof packing 11a, as shown in FIG. 4 and FIG. 5. Here, this protective glass 11 is attached to the inner surface of the attachment recess section 8a, or more specifically, the ceiling surface of the attachment recess section 8a with a first two-sided adhesive tape 11b. As a result, this protective glass 11 is structured to be pressed onto the skin of an arm together with the projection section 8 of the back cover 5 when the case 1 is worn on the arm by the bands 2.

In this embodiment, in an area between the attachment recess section 8a of the projection section 8 and the arrangement recess sections 9 of the back cover 5, a through hole 5a which connects these sections is provided, as shown in FIG. 4 and FIG. 5. Accordingly, the first two-sided adhesive tape 11b is formed in a substantially ring shape whose inner diameter is greater than that of the through hole 5a. That is, this first two-sided adhesive tape 11b is structured to be provided on the ceiling surface of the attachment recess section 8a and positioned around the outer circumference of the through hole 5a so that the through hole 5a is not closed.

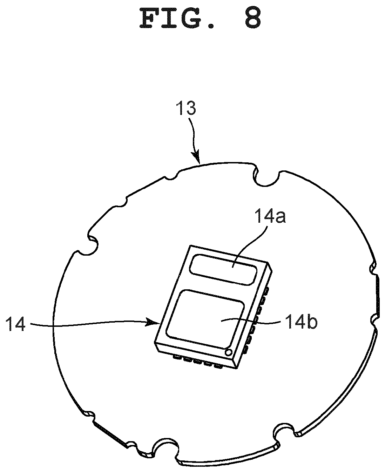

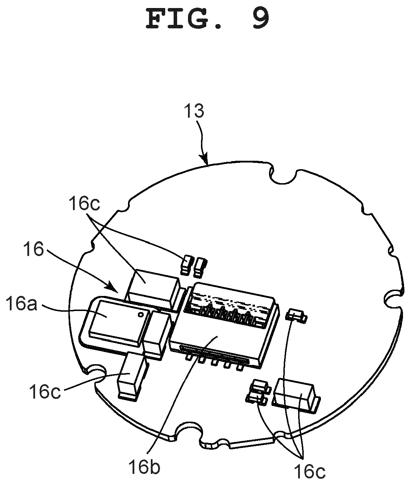

The circuit board 13 has a substantially circular plate shape and is made of a hard synthetic resin such as epoxy resin or glass epoxy resin, as shown in FIG. 4 and FIG. 5. On the undersurface (the surface shown in FIG. 8) of the circuit board 13, that is, on the back surface of the circuit board 13, the optical sensor 14 is provided, as shown in FIG. 8. Also, on the upper surface (the surface shown in FIG. 9) of the circuit board 13, that is, on the front surface of the circuit board 13, the various types of electronic components 16 are provided, as shown in FIG. 9.

These various types of electronic components 16 includes an Integrated Circuit (IC) chip 16a which electrically controls the optical sensor 14, a connector 16b to which a flexible wiring substrate (not shown) is connected, and other plural components 16c which are necessary for the detection device 10 to detect biological information, as shown in FIG. 9.

The optical sensor 14 and the various types of electronic components 16 described above are attached and electrically connected to a plurality of circuit patterns 13a on the upper surface and undersurface of the circuit board 13 by soldering, as shown in FIG. 4 and FIG. 5. This plurality of circuit patterns 13a, that is, the circuit pattern 13a on the upper surface and the circuit pattern 13a on the undersurface are electrically connected by a plurality of through holes (not shown) provided in the circuit board 13.

The circuit board 13 is structured such that the flexible wiring substrate (not shown) is connected to the connector 16b of the electronic components 16 in the state shown in FIG. 3, and electrically connected to the circuit section of the module in the case 1 through connection holes in the insulation sheet (not shown).

The optical sensor 14, which detects pulses, has a substantially quadrangular plate shape and is provided on a substantially center portion of the undersurface (the surface shown in FIG. 8) of the circuit board 13, as shown in FIG. 8. This optical sensor 14 is structured such that it is arranged in the through hole 5a between the attachment recess section 8a of the projection section 8 of the back cover 5 and the arrangement recess section 9 of the back cover 5 and its undersurface comes in close contact with the upper surface of the protective glass 11, as shown in FIG. 4 and FIG. 5.

That is, the optical sensor 14 is formed such that its thickness is greater than the axial length (depth) of the through hole 5a, as shown in FIG. 4 and FIG. 5. As a result, when the optical sensor 14 is arranged in the through hole 5a with it being attached to the undersurface of the circuit board 13, the lower part of the optical sensor 14 projects under the through hole 5a. Accordingly, when the upper surface of the protective glass 11 is pressed against the undersurface of the optical sensor 14, the circuit board 13 is pressed upward from the bottom of the arrangement recess section 9.

Also, the optical sensor 14 has a light emitting area 14a and a light receiving area 14b, as shown in FIG. 8. The light emitting area 14a, which includes a light emitting element such as a Light Emitting Diode (LED), is structured to emit light of a specific wavelength, such as light of the green wavelength. The light receiving area 14b, which includes a light receiving element such as a Photo Diode (PD), is structured to receive reflected light derived by light from the light emitting area 14a being applied to and reflecting on an arm, and convert the reflected light into an electric signal.

That is, the optical sensor 14 is structured such that, when light of a specific wavelength is emitted from the light emitting area 14a of FIG. 8 to an arm with the case 1 of FIG. 2 being worn on the arm by the bands 2, part of the emitted light is absorbed into blood in blood vessels, and the remaining light is reflected on the arm and received by the light receiving area 14b.

Here, the amount of blood in the blood vessels varies every time the heart beats, and therefore the amount of reflected light resulting from the emitted light varies in accordance with the variation in the amount of blood. The above-described optical sensor 14 is structured to receive a varied amount of reflected light by the light receiving area 14b shown in FIG. 8, convert it into an electric signal, and thereby detect a pulse.

On the other hand, the holder 12 has a substantially circular plate shape and is made of a hard synthetic resin, as shown in FIG. 3 to FIG. 7. This holder 12 is formed such that its thickness is sufficiently greater than the thickness of the circuit board 13 and substantially equal to the depth of the arrangement recess section 9 formed in the inner surface of the back cover 5. Also, this holder 12 is structured such that it is provided with a board housing section 12c where the circuit board 13 is arranged, and the circuit board 13 is arranged in this board housing section 12c without protruding downward.

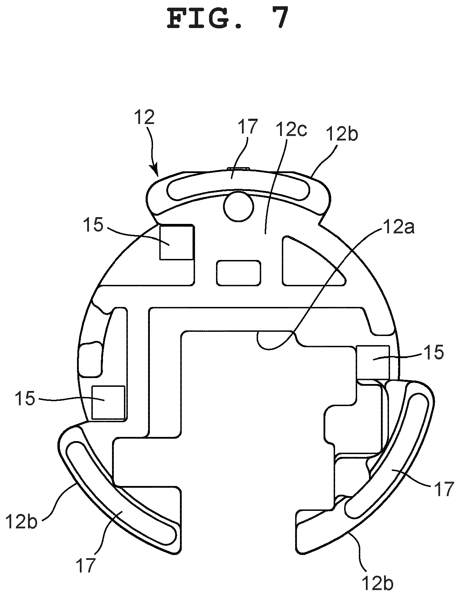

Outer circumferential portions 12b of this holder 12 downwardly project lower than the circuit board 13, and the undersurfaces of these projecting outer circumferential portions 12b are attached to the bottom of the arrangement recess section 9 of the back cover 5 with second two-sided adhesive tapes 17, whereby the holder 12 is fixed in the arrangement recess section 9 of the back cover 5, as shown in FIG. 4 to FIG. 7. In this embodiment, the second two-sided adhesive tapes 17 are applied to plural undersurface areas of the outer circumferential portions 12b of the holder 12, or more specifically, three areas including an area between the eleven o'clock side and the one o'clock side, an area between the three o' clock side and the five o' clock side, and an area between the seven o'clock side and the nine o'clock side, as shown in FIG. 7.

Also, this holder 12 is provided with an opening section 12a corresponding to the optical sensor 14 and the various types of electronic components 16 provided on the circuit board 13, as shown in FIG. 4 to FIG. 7. More specifically, the opening section 12a is a substantially quadrangular hole formed by part of the holder 12 from its substantially center portion to its outer circumferential rim portion on the six o' clock side being cut out.

As a result of this structure, when the holder 12 is attached to the bottom of the arrangement recess section 9 of the back cover 5 with the circuit board 13 being arranged in the board housing section 12c in the holder 12, the various types of electronic components 16 provided on the upper surface of the circuit board 13 are arranged in the opening section 12a of the holder 12, and the optical sensor 14 provided on the undersurface of the circuit board 13 is arranged corresponding to the opening section 12a of the holder 12, as shown in FIG. 3 to FIG. 7.

The energizing members 15 are made of cushioning material, as shown in FIG. 4 and FIG. 7. However, they may be spring members such as coil springs or plate springs. Each of the energizing members 15 is structured to force the circuit board 13 toward the protective glass 11, that is, the bottom of the arrangement recess section 9 of the back cover 5. These energizing members 15 are arranged in compressed states in areas between the undersurface of the holder 12 and the upper surface of the circuit board 13 while avoiding an area corresponding to the optical sensor 14 and the various types of electronic components 16 provided on the circuit board 13.

That is, on the inner surface of the board housing section 12c of the holder 12, these energizing members 15 are provided in a plurality of areas, or more specifically, three areas that are located around the outer periphery of the opening section 12a on a substantially three o' clock side, a substantially nine o'clock side, and a substantially eleven o'clock side, and do not include areas corresponding to the second two-sided adhesive tapes 17 on the holder 12 and the area corresponding to the optical sensor 14 and the various types of electronic components 16 provided on the circuit board 13, as shown in FIG. 7.

As a result of this structure, while being compressed by the holder 12 fixed in the arrangement recess section 9 of the back cover 5, the energizing members 15 force the circuit board 13 toward the bottom of the arrangement recess section 9 of the back cover 5 such that the circuit board 13 is evenly pressed with balance, and thereby press the undersurface of the optical sensor 14 on the circuit board 13 against the upper surface of the protective glass 11 such that the undersurface of the optical sensor 14 is evenly pressed with balance and comes in close contact with the upper surface of the protective glass 11, as shown in FIG. 4 and FIG. 7. In this embodiment, the fitting force of the protective glass 11 with respect to the attachment recess section 8a of the back cover 5 is greater than the energizing force of the energizing members 15.

As a result, the circuit board 13 is structured to be held between the protective glass 11 and the holder 12 when the undersurface of the optical sensor 14 is pressed against the upper surface of the protective glass 11 by the energizing force of the energizing members 15 in the compressed states, and thereby arranged in a floating state in the arrangement recess section 9 without coming in contact with the bottom surface of the arrangement recess section 9 and the inner surface of the board housing section 12c of the holder 12, as shown in FIG. 4.

Here, since the thickness of the optical sensor 14 in a vertical direction is greater than the axial length (depth) of the through hole 5a provided between the attachment recess section 8a in the projection section 8 of the back cover 5 and the arrangement recess section 9 of the back cover 5, the lower part of the optical sensor 14 downwardly protrudes from the through hole 5a when the optical sensor 14 is inserted into the through hole 5a, as shown in FIG. 4 and FIG. 5. As a result of this structure, the undersurface of the optical sensor 14 is pressed by the upper surface of the protective glass 11, whereby the circuit board 13 is pressed above the bottom of the arrangement recess section 9.

As a result, the circuit board 13 is structured to be held between the protective glass 11 and the holder 12 and arranged in the floating state in the board housing section 12c of the holder 12 in the arrangement recess section 9 without its undersurface coming in contact with the bottom surface of the arrangement recess section 9 and its upper surface coming in contact with the inner surface of the board housing section 12c of the holder 12, as shown in FIG. 4.

Next, the mechanism of this body-worn device is described.

When this body-worn device is to be used, first, the case 1 is worn on an arm by the bands 2 with the back cover 5 of the case 1 being arranged on the arm. Here, the projection section 8 of the back cover 5 is pressed against and comes in close contact with the skin of the arm with the protective glass 11 on the projection section 8 being pressed onto the skin.

In this state, one of the plurality of push button switches 3 on the case 1 is operated to switch the current mode to a pulse detection mode, whereby the detection device 10 provided in the back cover 5 starts pulse detection. In this detection, light of a specific wavelength, such as light of the green wavelength, is emitted from the light emitting area 14a of the optical sensor 14 of the detection device 10, and applied to the skin of the arm through the protective glass 11.

Here, since the protective glass 11 has been pressed onto and is in close contact with the skin of the arm, external light from outside the case 1 is blocked and only the light of the specific wavelength from the light emitting area 14a is applied to the skin of the arm. Part of this applied light is absorbed into blood in blood vessels under the skin and the remaining light is reflected. This reflected light is received by the light receiving area 14b of the optical sensor 14 through the protective glass 11 and converted into an electrical signal.

Here, blood in the blood vessels flows with pulses. Accordingly, the amount of blood therein varies every time the heart beats. Thus, the amount of reflected light resulting from the emitted light of the specific wavelength varies in accordance with the variation in the amount of blood, and a varied amount of reflected light is received by the light receiving area 14b of the optical sensor 14 through the protective glass 11. This light received by the light receiving area 14b is converted into an electrical signal, and a pulse is measured by an integrated circuit chip 15a of the electronic components 16 on the circuit board 13 on the basis of the electrical signal.

Also, here, the circuit board 13 has been forced toward the protective glass 11 by the energizing members 15 in the compressed states, and the undersurface of the optical sensor 14 provided on the circuit board 13 is in close contact with the upper surface of protective glass 11. Accordingly, in the pulse detection by the detection device 10, the light of the specific wavelength emitted from the light emitting area 14a of the optical sensor 14 is prevented from being reflected on the upper surface of the protective glass 11. That is, light reflection thereon can be suppressed.

More specifically, if the light of the specific wavelength emitted from the light emitting area 14a is reflected on the upper surface of the protective glass 11, this reflected light is received by the light receiving area 14b as noise, or in other words, as light other than that reflected on the skin of the arm. By this light reflection on the upper surface of the protective glass 11 being suppressed, the light emitted from the light emitting area 14a can be efficiently applied to the skin of the arm through the protective glass 11, which enhances the detection performance.

In this detection device 10, when the undersurface of the optical sensor 14 is pressed against the upper surface of the protective glass 11 by the energizing force of the energizing members 15, the circuit board 13 is held between the protective glass 11 and the holder 12 and thereby arranged in the floating state in the arrangement recess section 9 without coming in contact with the bottom surface of the arrangement recess section 9 and the inner surface of the board housing section 12c of the holder 12.

As a result of this structure, in this detection device 10, the optical sensor 14 provided on the circuit board 13 is evenly pressed against the protective glass 11 with balance by the energizing force of the energizing members 15. In addition, in the case of this detection device 10, when the case 1 is subjected to an external impact, this impact is buffered by the energizing members 15, and therefore the circuit board 13 is not affected by the impact. By this structure as well, the detection performance of the detection device 10 is enhanced.

In Japanese Patent Application Laid-Open (Kokai) Publication No. JP 2005-270543 A described above, the detection device for detecting biological information such as a pulse is disclosed which is provided on the back cover attached to the lower part of the case that is called a housing and worn on a part of a body such as an arm.

This detection device disclosed in Japanese Patent Application Laid-Open (Kokai) Publication No. JP 2005-270543 A has a structure in which a protective glass that is a transparent section is fitted into the back cover, a flexible substrate having an optical sensor is attached to the inner surface of the back cover in a manner to be positioned opposing the protective glass, and the optical sensor of the flexible substrate is positioned close to the protective glass by the resilience of the flexible substrate.

However, this detection device has a problem in that, because of the structure where the flexible substrate is attached to the back cover and the optical sensor is positioned close to the protective glass by the resilience of the flexible
substrate, the contact state of the optical sensor with respect to the protective glass is unstable, and therefore it is difficult to reliably and favorably bring the optical sensor into close contact with the protective glass.

In contrast, the detection device 10 in the body-worn device of the present embodiment includes the back cover 5 which is an exterior member and having the protective glass 11 that is a light transmissive transparent section, the holder 12 which is attached to the back cover 5 in a manner to be positioned opposing the protective glass 11, the circuit board 13 which is arranged between the back cover 5 and the holder 12, the optical sensor 14 which is provided on one surface of the circuit board 13 opposing the protective glass 11, that is, the undersurface of the circuit board 13 and detects light of a specific wavelength, and the energizing members 15 which are arranged between the holder 12 and the circuit board 13 and force the circuit board 13 and the optical sensor 14 toward the protective glass 11, whereby the optical sensor 14 can be favorably brought into close contact with the protective glass 11.

More specifically, in this detection device 10, the circuit board 13 arranged between the back cover 5 and the holder 12 is forced toward the protective glass 11 by the energizing members 15 and, by the energizing force of these energizing members 15, presses the optical sensor 14 against the protective glass 11. As a result of this structure, the optical sensor 14 can be reliably and favorably brought into close contact with the protective glass 11 in a stable state.

In this detection device 10 of the present embodiment, the optical sensor 14 includes the light emitting area 14a and the light receiving area 14b. Light emitted from the light emitting area 14a is applied to a part of a body such as an arm, and reflected light resulting from this applied light is received by the light receiving area 14b, whereby biological information such as a pulse is detected.

More specifically, in the case of this detection device 10, light of a specific wavelength, such as light of the green wavelength, is emitted from the light emitting area 14a of the optical sensor 14 and applied to the skin of an arm. Part of this emitted light is absorbed into blood in blood vessels under the skin, and the remaining light is reflected, received by the light receiving area 14b of the optical sensor 14, and converted into an electric signal, whereby a pulse is detected.

Here, blood in the blood vessels flows with pulses. Accordingly, the amount of blood therein varies every time the heart beats. Thus, the amount of reflected light resulting from the emitted light of the specific wavelength varies in accordance with the variation in the amount of blood, and a varied amount of reflected light is received by the light receiving area 14b of the optical sensor 14 in the detection device 10. This reflected light received by the light receiving area 14b is converted into an electrical signal, and a pulse is measured by the integrated circuit chip 15a of the electronic components 16 on the circuit board 13 on the basis of the electrical signal.

Also, in this detection device 10, the optical sensor 14 is pressed against the protective glass 11 by the energizing force of the energizing members 15 forcing the circuit board 13, whereby the optical sensor 14 is reliably and favorably brought into close contact with the protective glass 11 in the stable state. Accordingly, in this detection device 10, light reflection on the upper surface of the protective glass 11 when light emitted by the optical sensor 14 passes through the protective glass 11, that is, reflected light to be taken as noise can be suppressed, which enhances the detection performance.

That is, in this detection device 10, since the undersurface of the optical sensor 14 is in close contact with the upper surface of the protective glass 11, light reflection on the upper surface of the protective glass 11 when light emitted by the light emitting area 14a of the optical sensor 14 passes through the protective glass 11, that is, reflected light to be taken as noise is suppressed, whereby the light emitted by the light emitting area 14a efficiently passes through the protective glass 11 and is favorably applied to the skin of an arm.

As a result of this structure, in this detection device 10, a situation can be prevented in which, when light reflected on a part of a body such as an arm after passing through the protective glass 11 is to be received by the optical sensor 14, light reflected on the upper surface of the protective glass 11 and existing as noise, that is, light other than that reflected on the skin of the arm is received. Thus, the detection performance of the detection device 10 is further enhanced.

Also, in this detection device 10, the circuit board 13 is arranged in the floating state where it is not in contact with the back cover 5 that is an exterior member and the holder 12. As a result of this structure, the optical sensor 14 provided on the circuit board 13 is evenly pressed against the protective glass 11 with balance by the energizing force of the energizing members 15. In addition, when the case 1 is subjected to an external impact, this impact is buffered by the energizing members 15, so that the circuit board 13 can be prevented from being affected by the impact. By this structure as well, the detection performance of the detection device 10 is enhanced.

That is, in this detection device 10, the circuit board 13 held between the protective glass 11 and the holder 12 with the undersurface of the optical sensor 14 being pressed against the upper surface of the protective glass 11 by the energizing force of the energizing members 15 is in the floating state in the arrangement recess section 9 without being in contact with the bottom surface of the arrangement recess section 9 and the inner surface of the board housing section 12c of the holder 12.

As a result of this structure, in this detection device 10, the optical sensor 14 provided on the circuit board 13 is evenly pressed against the protective glass 11 with balance by the energizing force of the energizing members 15 and, even if the case 1 is subjected to an external impact, this impact is buffered by the energizing members 15, so that the circuit board 13 can be prevented from being affected by the impact.

Also, in the case of this detection device 10, the energizing members 15 are made of cushioning material, and arranged on outer circumferential areas on the other surface of the circuit board 13, that is, the upper surface of the circuit board 13 while avoiding the area corresponding to the optical sensor 14. As a result of this structure, a portion of the circuit board 13 corresponding to the optical sensor 14 is not pressed when the energizing members 15 force the circuit board 13, whereby the circuit board 13 can be favorably pressed by the energizing force of the energizing members 15 and the optical sensor 14 can be favorably pressed against the protective glass 11.

That is, in this detection device 10, the energizing members 15 are arranged on the plurality of outer circumferential areas on the upper surface of the circuit board 13, or more specifically, three areas on a substantially three o'clock side, a substantially nine o'clock side, and a substantially eleven o' clock side, whereby the circuit board 13 excluding its portion corresponding to the optical sensor 14 and the various types of electronic components 16 provided on the circuit board 13 is evenly pressed with balance by the energizing force of the energizing members 15. As a result of this structure, the optical sensor 14 can be further reliably and favorably brought into close contact with the protective glass 11 in the stable state.

Here, in this detection device 10, the energizing members 15 are arranged in the compressed states in the areas between the holder 12 and the circuit board 13. As a result, by the energizing force of these energizing members 15, the circuit board 13 is further evenly pressed with balance, whereby the optical sensor 14 can be further reliably and favorably brought into close contact with the protective glass 11 in the stable state.

Also, in this detection device 10, the opening section 12a corresponding to the optical sensor 14 is formed in the holder 12. By this opening section 12a of the holder 12, the portion of the circuit board 13 corresponding to the optical sensor 14 is not pressed even when the circuit board 13 is forced by the energizing members 15 arranged between the holder 12 and the circuit board 13, whereby the optical sensor 14 can be favorably arranged on the protective glass 11 by the energizing members 15 without being affected by the holder 12.

In the case of this detection device 10, in the opening section 12a, the electronic components 16 provided on the other surface of the circuit board 13, that is, the upper surface of the circuit board 13 while avoiding the energizing members 15 is arranged. As a result of this structure, when the circuit board 13 is arranged in the holder 12, the electronic components 16 of the circuit board 13 are arranged in the opening section 12a of the holder 12, and thereby can be prevented from coming in contact with the holder 12.

In the above-described embodiment, as the transparent section which is light transmissive, the protective glass 11 is used. However, the present invention is not limited thereto, and a light transmissive plate made of a synthetic resin, such as Polymethyl methacrylate (PMMA) or Polycarbonate (PC), may be adopted. Also, for example, a transparent material provided with a film through which light of a specific wavelength passes or a filter layer such as an evaporated film may be adopted as the transparent section.

Also, in the above-described embodiment, the energizing members 15 are arranged on the plurality of outer circumferential areas on the upper surface of the circuit board 13. However, the present invention is not limited thereto, and the energizing members 15 may be arranged on any portion of the upper surface of the circuit board 13 as long as it is not a portion corresponding to the optical sensor 14 and the various types of electronic components 16 mounted on the circuit board 13.

Moreover, in the above-described embodiment, the energizing members 15 are made of cushioning material. However, the present invention is not limited thereto, and spring members such as extension coil springs may serve as the energizing members 15. In that case, a structure in which the protective glass 11 that is a transparent section is pulled toward the optical sensor 14 side, a structure in which the circuit board 13 and the optical sensor 14 are pulled toward the protective glass 11, or a structure in which the holder 12 is pulled toward the circuit board 13 may be adopted.

Furthermore, in the above-described embodiment, the undersurfaces of the outer circumferential portions 12b of the holder 12 are attached to the bottom of the arrangement recess section 9 of the back cover 5 with the second two-sided adhesive tapes 17. However, the present invention is not limited thereto, and a structure may be adopted in which the holder 12 is attached to the arrangement recess section 9 of the back cover 5 with fastening members such as screw members.

Still further, in the above-described embodiment, the body-worn device is worn on an arm and used. However, the present invention is not limited thereto, and it may be worn on a part of a body other than arms.

## Claims

1. A detection device (10) comprising:
a back cover (5) which is provided with a light transmissive transparent portion (11);
a holder (12)
positioned opposing the transparent portion (11);
a circuit board ( 13 );
an optical sensor (14) which is provided on one surface of the circuit board (13) opposing the transparent portion (11) to detect light; and
resilient energizing members (15);
wherein the circuit board (13) is arranged between the back cover (5) and the holder (12);
wherein the energizing members (15) are made of cushioning material or are spring members such as coil springs or plate springs, and arranged on an outer circumferential area which is on another surface opposite to the one surface of the circuit board (13) and does not include an area corresponding the optical sensor (14); and
wherein the energizing members (15) are arranged in compressed states in areas between the undersurface of the holder (12) and the upper surface of the circuit board (13),thereby forcing the circuit board (13) and the optical sensor (14) toward the transparent portion (11) **characterized in that** the holder (12) is attached to an arrangement recess section of the back cover.

2. The detection device (10) according to claim 1, wherein the optical sensor (14) has a light emitting area (14a) and a light receiving area (14b).

3. The detection device (10) according to claim 1 or 2, wherein the optical sensor (14) is pressed against the transparent portion (11) by energizing force of the energizing members (15) forcing the circuit board (13).

4. The detection device (10) according to any one of claims 1 to 3, wherein the circuit board (13) is arranged in a floating state where the circuit board (13) is not in contact with the back cover (5) and the holder (12).

5. The detection device (10) according to any one of claims 1 to 4, wherein the holder (12) has an opening portion (12a) provided corresponding to the optical sensor (14).

6. The detection device (10) according to claim 5, wherein an electronic component (16) provided on another surface of the circuit board (13) while avoiding the energizing members (15) is arranged in the opening portion (12a).

7. The detection device (10) according to any one of claims 1 to 6, wherein light of a specific wavelength passes through the transparent portion (11).

8. A body-worn device comprising the detection device (10) according to any one of claims 1 to 7.

## Patentansprüche

1. Erfassungsvorrichtung (10), umfassend:
eine Rückwand (5), die mit einem lichtdurchlässigen transparenten Abschnitt (11) ausgestattet ist;
einen Halter (12), der dem transparenten Abschnitt (11) gegenüberliegt;
eine Leiterplatte (13);
einen optischen Sensor (14), der auf einer dem transparenten Abschnitt (11) gegenüberliegenden Fläche der Leiterplatte (13) angeordnet ist, um Licht zu erfassen; und
elastische Erregerelemente (15);
wobei die Leiterplatte (13) zwischen der Rückwand (5) und dem Halter (12) angeordnet ist;
wobei die Erregerelemente (15) aus einem Dämpfungsmaterial bestehen oder Federelemente wie Schraubenfedern oder Tellerfedern sind und in einem Umfangsbereich angeordnet sind, der sich auf einer anderen Fläche befindet, die der einen Fläche der Leiterplatte (13) gegenüberliegt und keinen Bereich umfasst, der dem optischen Sensor (14) entspricht; und
wobei die Erregerelemente (15) in komprimierten Zuständen in Bereichen zwischen der Unterseite des Halters (12) und der Oberseite der Leiterplatte (13) angeordnet sind, wodurch die Leiterplatte (13) und der optische Sensor (14) in Richtung des transparenten Abschnitts (11) gedrückt werden, **dadurch gekennzeichnet, dass** der Halter (12) an einem Anordnungsaussparungsabschnitt der Rückwand angebracht ist.

2. Erfassungsvorrichtung (10) nach Anspruch 1, wobei der optische Sensor (14) einen Lichtausstrahlbereich (14a) und einen Lichtempfangsbereich (14b) aufweist.

3. Erfassungsvorrichtung (10) nach Anspruch 1 oder 2, wobei der optische Sensor (14) durch die Erregerkraft der Erregerelemente (15), welche die Leiterplatte (13) antreiben, gegen den transparenten Abschnitt (11) gedrückt wird.

4. Erfassungsvorrichtung (10) nach einem der Ansprüche 1 bis 3, wobei die Leiterplatte (13) in einem schwebenden Zustand angeordnet ist, in dem die Leiterplatte (13) nicht in Kontakt mit der Rückwand (5) und dem Halter (12) steht.

5. Erfassungsvorrichtung (10) nach einem der Ansprüche 1 bis 4, wobei der Halter (12) einen Öffnungsabschnitt (12a) aufweist, der entsprechend dem optischen Sensor (14) angeordnet ist.

6. Erfassungsvorrichtung (10) nach Anspruch 5, wobei eine elektronische Komponente (16), die auf einer anderen Fläche der Leiterplatte (13) angeordnet ist, während sie die Erregerelemente (15) vermeidet, im Öffnungsabschnitt (12a) angeordnet ist.

7. Erfassungsvorrichtung (10) nach einem der Ansprüche 1 bis 6, wobei Licht einer bestimmten Wellenlänge durch den transparenten Abschnitt (11) fällt.

8. Am Körper getragene Vorrichtung, umfassend die Erfassungsvorrichtung (10) nach einem der Ansprüche 1 bis 7.

## Revendications

1. Dispositif de détection (10) comprenant :
un capot arrière (5) qui est équipé d'une partie transparente de transmission de lumière (11) ;
un support (12) positionné faisant face à la partie transparente (11) ;
une carte de circuits (13) ;
un capteur optique (14) qui est disposé sur une surface de la carte de circuits (13) faisant face à la partie transparente (11) pour détecter une lumière ; et
des éléments de charge électrique souples (15) ;
dans lequel
la carte de circuits (13) est disposée entre le capot arrière (5) et le support (12) ;
dans lequel les éléments de charge électrique (15) sont constitués d'un matériau amortissant ou sont des éléments de ressort tels que des ressorts hélicoïdaux ou des ressorts à lames, et disposés sur une zone circonférentielle externe qui est placée sur une autre surface opposée à l'une surface de la carte de circuits (13) et n'inclut pas une zone correspondant au capteur optique (14) ; et
dans lequel les éléments de charge électrique (15) sont disposés dans des états comprimés dans des zones présentes entre la surface intérieure du support (12) et la surface supérieure de la carte de circuits (13), forçant ainsi la carte de circuits (13) et le capteur optique (14) vers la partie transparente (11) **caractérisé en ce que** le support (12) est fixé à une section en cavité d'agencement du capot arrière.

2. Le dispositif de détection (10) selon la revendication 1, dans lequel le capteur optique (14) présente une zone émission de lumière (14a) et une zone de réception de lumière (14b).

3. Le dispositif de détection (10) selon la revendication 1 ou 2, dans lequel le capteur optique (14) est appuyé contre la partie transparente (11) par une force de charge électrique des éléments de charge électrique (15) forçant la carte de circuits (13).

4. Le dispositif de détection (10) selon l'une quelconque des revendications 1 à 3, dans lequel la carte de circuits (13) est disposée dans un état flottant où la carte de circuits (13) n'est pas en contact avec le capot arrière (5) et le support (12).

5. Le dispositif de détection (10) selon l'une quelconque des revendications 1 à 4, dans lequel le support (12) dispose d'une partie d'ouverture (12a) correspondant au capteur optique (14).

6. Le dispositif de détection (10) selon la revendication **5,** dans lequel un composant électronique (16) disposé sur une autre surface de la carte de circuits (13) tout en évitant les éléments de charge électrique (15) est disposé dans la partie d'ouverture (12a).

7. Le dispositif de détection (10) selon l'une quelconque des revendications 1 à 6, dans lequel une lumière d'une longueur d'onde spécifique traverse la partie transparente (11).

8. Dispositif porté sur le corps comprenant le dispositif de détection (10) selon l'une quelconque des revendications 1 à 7.
